# EUROPEAN PATENT APPLICATION

(11) **EP 4 406 422 A2**
(43) Date of publication of application: **31.07.2024**
(21) Application number: 24182690.8
(22) Date of filing: 16.10.2020
(51) Int. Cl.: A23L 33/18

(54) **INFANT FORMULA**

(30) Priority: 17.10.2019 EP 19203860
(62) Divisional of application: 20790005.1
(71) Applicant: Société des Produits Nestlé S.A., 1800 Vevey (CH)
(72) Inventor: KUSLYS, Martinas, Jurgis, Grosshoechstetten (CH); HEINE, Ralf, Günter, Berlin (DE)
(74) Representative: Elkington and Fife LLP

(57) **Abstract**

An infant formula for use in reducing the occurrence of, or preventing, an infection in an infant, wherein the infant formula is an extensively hydrolysed infant formula (eHF) or an amino acid-based infant formula (AAF), wherein the infant formula comprises protein, carbohydrate and fat, wherein the eHF comprises about 2.4 g or less protein per 100 kcal, or the AAF comprises about 2.9g or less protein per 100kcal, wherein the infant formula further comprises 2'-fucosyllactose (2'FL) and/or lacto-N-neotetraose (LNnT), and wherein about 30% or less by weight of the fat is medium chain triglycerides (MCTs).

## Description

### FIELD OF THE INVENTION

The present invention relates to extensively hydrolysed infant formulas (eHFs) and amino acid-based infant formulas (AAFs) comprising a reduced amount of protein and comprising human milk oligosaccharide (HMOs), for use in reducing the occurrence of an infection or preventing an infection. The formula may be used in an infant with cow's milk protein allergy.

### BACKGROUND TO THE INVENTION

Cow's milk protein (CMP) is the leading cause of food allergy in infants, affecting 2-3% children worldwide. Most children with CMP-allergy (CMPA) have two or more symptoms: 50-70% have skin symptoms; 50-60% have gastrointestinal symptoms; and 20-30% have airway symptoms. Severe and life-threatening symptoms may occur in 10% of children. (Nutten, 2018. EMJ Allergy Immunol, 3(1), pp. 50-59).

Human breast milk and breast feeding are considered to be the optimal form of nutrition for healthy infants during the first months of life. Breast milk remains the gold standard for feeding infants with CMPA. The European Society for Paediatric Gastroenterology, Hepatology and Nutrition (ESPGHAN) recommends that CMPA is best treated in breast-fed infants by complete elimination of cow's milk from the mother's diet (Koletzko, S., et al., 2012. Journal of pediatric gastroenterology and nutrition, 55(2), pp.221-229).

Specialty infant formulas are recommended when breastfeeding is not possible. ESPGHAN recommends that for non-breast-fed infants with CMPA, formulas based on extensively hydrolysed proteins are used, with proven efficacy in infants with CMPA. In infants with extremely severe or life-threatening symptoms an amino acid-based infant formula (AAF) may be considered as the first choice (Koletzko, S., et al., 2012. Journal of pediatric gastroenterology and nutrition, 55(2), pp.221-229).

Extensively hydrolyzed infant formulas (eHFs) and AAFs may have lower absorption of nitrogen than full protein formulas or human milk (Rigo, J., et al., 1995. European journal of clinical nutrition, 49, pp.S26-38). Thus, eHFs typically contain 2.6-2.8 g protein per 100 kcal, and AAFs typically contain 2.8-3.1 g protein per 100 kcal, to cover the needs of infants (Borschel, M., et al., 2018. Nutrients, 10(3), p.289).

Consumption of higher protein infant formula has, however, been associated with greater weight and body mass index at 2 years of age and higher circulating concentrations of plasma essential amino acids, insulin-like growth factor-1, and C-peptide, which can induce weight gain and adipogenic activity. Lower protein content may diminish the later risk of obesity (Totzauer, M., et al., 2018. Obesity, 26(7), pp.1203-1210).

Furthermore, infants with CMP-allergy (CMPA) may be at higher risk of infection. For example, infants with CMPA are known to have a higher prevalence of respiratory tract infections (Woicka-Kolejwa, K., et al., 2016. Advances in Dermatology and Allergology, 33(2), p.109). CMPA has also been associated with recurrent otitis media during childhood (Juntti, H., Acta Oto-Laryngologica, 119(8), pp.867-873).

### SUMMARY OF THE INVENTION

The inventors have surprisingly found that eHFs and AAFs comprising the human milk oligosaccharides (HMOs) 2'-fucosyllactose (2'FL) and/or lacto-N-neotetraose (LNnT), and with a reduced amount of protein, that have a protein content closer to that of human breast milk, supported appropriate growth and development of infants with CMPA, were safe and well-tolerated, and reduced the incidence of infection.

Accordingly, in one aspect the invention provides an infant formula for use in reducing the occurrence of, or preventing, an infection in an infant, wherein the infant formula is an extensively hydrolysed infant formula (eHF) or an amino acid-based infant formula (AAF), wherein the infant formula comprises protein, carbohydrate and fat, wherein the eHF comprises about 2.4 g or less protein per 100 kcal, or the AAF comprises about 2.9g or less protein per 100kcal, wherein the infant formula further comprises 2'-fucosyllactose (2'FL) and/or lacto-N-neotetraose (LNnT), and wherein about 30% or less by weight of the fat is medium chain triglycerides (MCTs).

In another related aspect the invention provides a method of reducing the occurrence of, or preventing, an infection in an infant, wherein the method comprises administering to the infant an infant formula, wherein the infant formula is an eHF or an AAF, wherein the infant formula comprises protein, carbohydrate and fat, wherein the eHF comprises about 2.4 g or less protein per 100 kcal, or the AAF comprises about 2.9g or less protein per 100kcal wherein the infant formula further comprises 2'-fucosyllactose (2'FL) and/or lacto-N-neotetraose (LNnT), and wherein about 30% or less by weight of the fat is medium chain triglycerides (MCTs).

In another aspect the invention provides an infant formula for use in treating an infection in an infant, wherein the infant formula is an eHF or an AAF, wherein the infant formula comprises protein, carbohydrate and fat, wherein the eHF comprises about 2.4 g or less protein per 100 kcal, or the AAF comprises about 2.9g or less protein per 100kcal wherein the infant formula further comprises 2'-fucosyllactose (2'FL) and/or lacto-N-neotetraose (LNnT), and wherein about 30% or less by weight of the fat is medium chain triglycerides (MCTs).

In one aspect the invention provides an AAF for use in reducing the occurrence of, or preventing, an infection in an infant, wherein the AAF comprises protein, carbohydrate and fat, wherein the AAF comprises about 2.8g or less protein per 100kcal, preferably 2.7g or less protein per 100kcal, more preferably 2.6 g or less protein per 100 kcal, wherein the AAF further comprises 2'-fucosyllactose (2'FL) and/or lacto-N-neotetraose (LNnT), and wherein about 30% or less by weight of the fat is medium chain triglycerides (MCTs). Suitably, the AAF may comprise 2.5 g or less protein per 100 kcal.

In another related aspect the invention provides a method of reducing the occurrence of, or preventing, an infection in an infant, wherein the method comprises administering to the infant an AAF, wherein the AAF comprises protein, carbohydrate and fat, wherein the AAF comprises about 2.8g or less protein per 100kcal, preferably 2.7g or less protein per 100kcal, more preferably 2.6 g or less protein per 100 kcal, wherein the AAF further comprises 2'-fucosyllactose (2'FL) and/or lacto-N-neotetraose (LNnT), and wherein about 30% or less by weight of the fat is medium chain triglycerides (MCTs). Suitably, the AAF may comprise 2.5 g or less protein per 100 kcal.

In another related aspect the invention provides a method of treating an infection in an infant, wherein the method comprises administering to the infant an infant formula, wherein the infant formula is an eHF or an AAF, wherein the infant formula comprises protein, carbohydrate and fat, wherein the infant formula comprises about 2.4 g or less protein per 100 kcal, wherein the infant formula further comprises 2'-fucosyllactose (2'FL) and/or lacto-N-neotetraose (LNnT), and wherein about 30% or less by weight of the fat is medium chain triglycerides (MCTs).

In another aspect the invention provides an AAF for use in treating an infection in an infant, wherein the AAF comprises protein, carbohydrate and fat, wherein the AAF comprises about 2.9 g or less protein per 100kcal, preferably about 2.8g or less protein per 100kcal, 2.7g or less protein per 100kcal, more preferably 2.6 g or less protein per 100 kcal, wherein the AAF further comprises 2'-fucosyllactose (2'FL) and/or lacto-N-neotetraose (LNnT), and wherein about 30% or less by weight of the fat is medium chain triglycerides (MCTs). Suitably, the AAF may comprise 2.5 g or less protein per 100 kcal.

In another related aspect the invention provides a method of treating an infection in an infant, wherein the method comprises administering to the infant an AAF, wherein the AAF comprises protein, carbohydrate and fat, wherein the AAF comprises about 2.9 g or less protein per 100kcal, preferably about 2.8g or less protein per 100kcal, 2.7g or less protein per 100kcal, more preferably 2.6 g or less protein per 100 kcal, wherein the AAF further comprises 2'-fucosyllactose (2'FL) and/or lacto-N-neotetraose (LNnT), and wherein about 30% or less by weight of the fat is medium chain triglycerides (MCTs). Suitably, the AAF may comprise 2.5 g or less protein per 100 kcal.

Preferably, the infant has cow's milk protein allergy.

The infection may be an ear infection, a respiratory infection, a gastrointestinal infection and/or a urinary tract infection. Preferably, the infection is a respiratory infection, such as an upper respiratory tract infection and/or a lower respiratory tract infection. Preferably, the infection is an ear infection, more preferably a middle ear infection.

The infant formula may comprise 2'FL and LNnT. The infant formula may comprise 0.5-3 g/L, 0.8-1.5 g/L, or about 1 g/L 2'FL, preferably, the infant formula comprises about 1 g/L 2'FL; and/or the infant formula may comprise 0.2-1 g/L, 0.5-0.8 g/L, or about 0.5 g/L LNnT, preferably the infant formula comprises about 0.5 g/L LNnT. Most preferably, the infant formula comprises about 1 g/L 2'FL and about 0.5 g/L LNnT.

The infant formula may comprise 1.8-2.4 g protein per 100 kcal, 2.1-2.3 g protein per 100 kcal, or 2.15-2.25 g protein per 100 kcal. Preferably the infant formula comprises about 2.2 g protein per 100 kcal.

About 30% or less by weight, about 25% or less by weight, 20% or less by weight, 15% or less by weight, 10% or less by weight, 5% or less by weight, or 1% or less by weight of the fat in the infant formula may be medium chain triglycerides (MCTs). Preferably, the infant formula comprises no added MCTs.

The infant formula may comprise 9-14 g carbohydrate per 100 kcal and/or 4.0-6.0 g fat per 100 kcal.

In one embodiment, the infant formula is an eHF. In one embodiment, the infant formula is an AAF. Preferably, the infant formula is an eHF.

At least about 95%, at least about 98%, at least about 99% or about 100% by weight of the peptides in the eHF may have a molecular mass of less than about 3000 Da. Preferably, there are no detectable peptides in the eHF about 3000 Da or greater in size.

At least about 90%, at least about 95%, at least about 98% or at least about 99% by weight of the peptides in the eHF may have a molecular mass of less than about 1500 Da. Preferably, at least about 99% of the peptides in the eHF have a molecular mass of less than about 1500 Da.

At least about 85%, at least about 90%, at least about 95%, at least about 98% or at least about 99% by weight of the peptides in the eHF may have a molecular mass of less than about 1200 Da. Preferably, at least 98% of the peptides by weight have a molecular mass of less than about 1200 Da.

At least about 45%, at least about 50%, 45-55%, or 50-54% by weight of the peptides in the eHF may be di- and tri-peptides. Preferably, about 51-53%, or more preferably, about 52% by weight of the peptides in the eHF are di- and tri-peptides.

At least about 45%, at least about 50%, 45-55%, or 50-54% by weight of the peptides in the eHF may have a molecular weight of between 240 and 600 Da. Preferably, about 51-53%, or more preferably about 52% by weight of the peptides in the eHF have a molecular weight of between 240 and 600 Da.

At least about 50%, at least about 60%, at least about 70%, at least about 80%, at least about 90%, or about 100% of the protein in the eHF may be whey protein. Preferably, the protein source is whey protein.

The eHF may comprise free amino acids. The free amino acids may be present in a concentration of 50% or less, 40% or less, 30% or less, or 25% or less by weight based on the total weight of amino acids. Preferably, the free amino acids are present in a concentration of 20-25%, 21-23%, or about 22% by weight based on the total weight of amino acids.

### DESCRIPTION OF DRAWINGS

**Figure 1** **- Non-inferiority: Weight gain per day**
   Treatment effect on weight gain at visit 4. Primary analysis shows that weight gain [g/d] of infants receiving Test formula was non-inferior to growth with the Control formula.

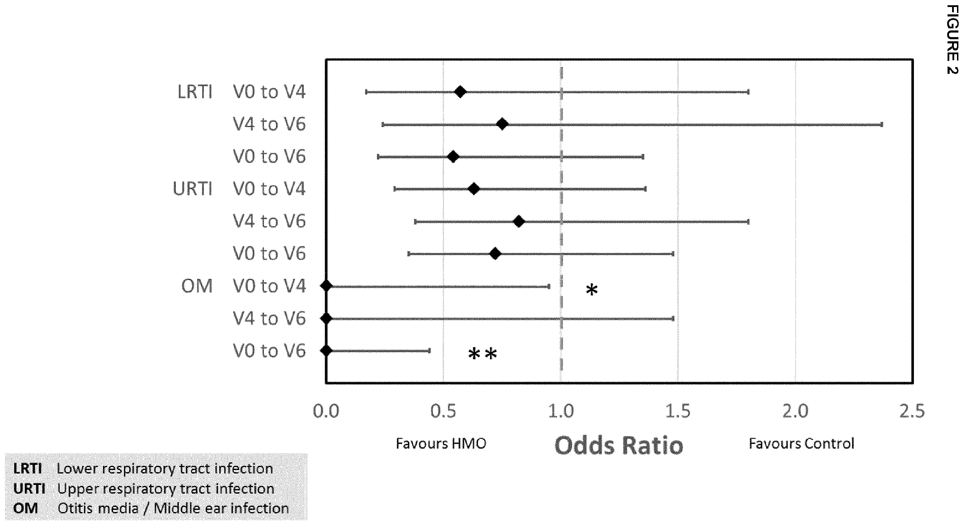

**Figure 2** **- Odds ratios: Respiratory and ear infections**
   Odds ratios for lower respiratory tract infection (LTRI); upper respiratory tract infection (UTRI); and otitis media/middle ear infection (OM) comparing groups fed Test formula (HMO) or Control Formula. The rate of OM was significantly lower in the group fed Test formula between V0-V4 and V0-V6.
**Figure 3** **- Relative risk reduction: Respiratory and ear infections**
   Relative risk reduction for lower respiratory tract infections; upper respiratory tract infections; and middle ear infections for infants fed Test formula. The relative risk reduction of middle ear infections was significant in the group fed Test formula.
**Figure 4****- Odds ratios: Medication use (antibiotics, antipyretics)**
   Odds ratios for use of antibiotics and antipyretics comparing groups fed Test formula (HMO) or Control formula. The use of antipyretics was significantly lower in the Test formula group between V4-V6.

### DETAILED DESCRIPTION OF THE INVENTION

Various preferred features and embodiments of the present invention will now be described by way of non-limiting examples.

It must be noted that as used herein and in the appended claims, the singular forms "a", "an", and "the" include plural referents unless the context clearly dictates otherwise.

The terms "comprising", "comprises" and "comprised of" as used herein are synonymous with "including" or "includes"; or "containing" or "contains", and are inclusive or open-ended and do not exclude additional, non-recited members, elements or steps. The terms "comprising", "comprises" and "comprised of" also include the term "consisting of".

As used herein the term "about" means approximately, in the region of, roughly, or around. When the term "about" is used in conjunction with a numerical value or range, it modifies that value or range by extending the boundaries above and below the numerical value(s) set forth. In general, the terms "about" and "approximately" are used herein to modify a numerical value(s) above and below the stated value(s) by 10%.

The publications discussed herein are provided solely for their disclosure prior to the filing date of the present application. Nothing herein is to be construed as an admission that such publications constitute prior art to the claims appended hereto.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range.

### Infant formula

The term "infant formula" may refer to a foodstuff intended for particular nutritional use by infants during the first year of life and satisfying by itself the nutritional requirements of this category of person, as defined in European Commission Regulation (EU) 2016/127 of 25 September 2015.

The infant formula of the present invention is an extensively hydrolysed infant formula (eHF) or an amino acid-based infant formula (AAF). Preferably, the infant formula is an eHF.

The term "extensively hydrolysed infant formula" or "eHF" may refer to an infant formula comprising extensively hydrolysed protein. The eHF may be a hypoallergenic infant formula which provides complete nutrition for infants who cannot digest intact CMP or who are intolerant or allergic to CMP.

The term "amino acid-based infant formula" or "AAF" may refer to an infant formula comprising only free amino acids as a protein source. The AAF may contain no detectable peptides. The AAF may be a hypoallergenic infant formula which provides complete nutrition for infants with food protein allergy and/or food protein intolerance. For example, the AAF may be a hypoallergenic infant formula which provides complete nutrition for infants who cannot digest intact CMP or who are intolerant or allergic to CMP, and who may have extremely severe or life-threatening symptoms and/or sensitisation against multiple foods.

A "hypoallergenic" composition is a composition which is unlikely to cause allergic reactions. Suitably, the infant formula of the invention is tolerated by more than 90% of infants with CMPA. This is in line with the guidance provided by the American Academy of Pediatrics (Committee on Nutrition, 2000. Pediatrics, 106(2), pp.346-349). Suitably, the infant formula of the invention may not contain peptides which are recognized by CMP-specific IgE e.g. IgE from subjects with CMPA.

Infants can be fed solely with the infant formula or the infant formula can be used as a complement of human milk.

The infant formula of the invention may be in the form of a powder or liquid.

The liquid may be, for example, a concentrated liquid infant formula or a ready-to-feed infant formula. The infant formula may be in the form of a reconstituted infant formula (i.e. a liquid infant formula that has been reconstituted from a powdered form). The concentrated liquid infant formula is preferably capable of being diluted into a liquid composition suitable for feeding an infant, for example by the addition of water.

In one embodiment, the infant formula is in a powdered form. The powder is capable of being reconstituted into a liquid composition suitable for feeding an infant, for example by the addition of water.

The infant formula may have an energy density of about 60-72 kcal per 100 mL, when formulated as instructed. Suitably, the infant formula may have an energy density of about 60-70 kcal per 100 mL, when formulated as instructed.

### Human milk oligosaccharides

The infant formula of the invention contains one or more human milk oligosaccharide (HMO).

Many different kinds of HMOs are found in the human milk. Each individual oligosaccharide is based on a combination of glucose, galactose, sialic acid (N-acetylneuraminic acid), fucose and/or N-acetylglucosamine with many and varied linkages between them, thus accounting for the enormous number of different oligosaccharides in human milk - over 130 such structures have been identified so far. Almost all of them have a lactose moiety at their reducing end while sialic acid and/or fucose (when present) occupy terminal positions at the non-reducing ends. HMOs can be acidic (e.g. charged sialic acid containing oligosaccharide) or neutral (e.g. fucosylated oligosaccharide).

The infant formula of the invention comprises 2'-fucosyllactose (2'FL) and/or lacto-N-neotetraose (LNnT).

The infant formula of the invention may comprise 2'FL. In some embodiments, there is no other type of fucosylated oligosaccharide than 2'FL, i.e. the infant formula of the invention comprises only 2'FL as fucosylated oligosaccharide.

The 2'FL may be produced by biotechnological means using specific fucosyltransferases and/or fucosidases either through the use of enzyme-based fermentation technology (recombinant or natural enzymes) or microbial fermentation technology. In the latter case, microbes may either express their natural enzymes and substrates or may be engineered to produce respective substrates and enzymes. Alternatively, 2'FL may be produced by chemical synthesis from lactose and free fucose.

The infant formula of the invention may comprise LNnT. In some embodiments, there is no other type of N-acetylated oligosaccharide than LNnT, i.e. the infant formula of the invention comprises only LNnT as N-acetylated oligosaccharide.

The LNnT may be synthesised chemically by enzymatic transfer of saccharide units from donor moieties to acceptor moieties using glycosyltransferases as described for example in US patent No. 5,288,637 and WO 96/10086. Alternatively, LNnT may be prepared by chemical conversion of Keto-hexoses (e.g. fructose) either free or bound to an oligosaccharide (e.g. lactulose) into N-acetylhexosamine or an N-acetylhexosamine-containing oligosaccharide as described in Wrodnigg, T.M.; Stutz, A.E. (1999) Angew. Chem. Int. Ed. 38:827-828. N-acetyllactosamine produced in this way may then be transferred to lactose as the acceptor moiety.

In some embodiments, the infant formula of the present invention comprises an oligosaccharide mixture that comprises 2'FL and/or LNnT. In a preferred embodiment, the infant formula of the present invention comprises an oligosaccharide mixture that consists of 2'FL and LNnT. The infant formula of the invention may comprise only 2'FL as fucosylated oligosaccharide and only LNnT as N-acetylated oligosaccharide.

2'FL can be present in the infant formula according to the present invention in a total amount of 0.5-3 g/L such as 0.8-1.5 g/L of the infant formula (when formulated as instructed). In some embodiments, 2'-fucosyllactose may be in a total amount of 0.85-1.3 g/L of the infant formula, such as 0.9-1.25 g/L or 0.9-1.1 g/L or 1-1.25 g/L or 1-1.2 g/L of the infant formula (when formulated as instructed). Preferably, the infant formula (when formulated as instructed) comprises about 1 g/L 2'-fucosyllactose.

LNnT can be present in the infant formula according to the present invention in a total amount of 0.2-1 g/L such as 0.5-0.8 g/L of the infant formula (when formulated as instructed). In some embodiments, LNnT may be in a total amount of 0.5-0.75 g/L or 0.5-0.7 g/L or 0.5-0.6 g/L of the infant formula (when formulated as instructed). Preferably, the infant formula (when formulated as instructed) comprises about 0.5 g/L LNnT.

These different ranges can all be combined together.

Therefore in one embodiment of the present invention, the infant formula (when formulated as instructed) comprises 2'FL and LNnT wherein:
(i) 2'FL is in a total amount of 0.8-1.5 g/L of the infant formula; and/or
(ii) LNnT is in a total amount of 0.5-0.8 g/L of the infant formula.

In another embodiment the infant formula of the present invention (when formulated as instructed) comprises 2'FL and LNnT wherein:
(i) 2'FL is in a total amount of 0.9-1.25 g/L of the infant formula; and/or
(ii) LNnT is in a total amount of 0.5-0.7 g/L of the infant formula.

In another embodiment the infant formula of the present invention (when formulated as instructed) comprises 2'FL and LNnT wherein:
(i) 2'FL is in a total amount of 1-1.2 g/L of the infant formula; and/or
(ii) LNnT is in a total amount of 0.5-0.6 g/L of the infant formula.

In a preferred embodiment, the infant formula of the present invention (when formulated as instructed) comprises about 1 g/L 2'FL and about 0.5 g/L LNnT.

The infant formula of the present invention may comprise 0.075-0.5 g/100kcal, 0.1-0.3 g/100kcal, or 0.12-0.25 g/100kcal 2'FL and about 0.03-0.15 g/100kcal, 0.05-0.12 g/100kcal, or 0.05-0.1 g/100kcal LNnT. Preferably, the infant formula of the present invention comprises about 0.15 g/100kcal 2'FL and about 0.075 g/100kcal LNnT.

The 2'FL and the LNnT comprised in the infant formula according to the invention are typically present in a ratio 2'FL: LNnT of from 2.0:0.54 to 2.0:2.26, such as 2.0:0.76 to 2.0:1.8 or 2.0:0.8 to 2.0:1.4. In a particularly advantageous embodiment, this ratio is 2.0:1 or around 2.0:1.

### Protein

The term "protein" includes peptides and free amino acids. The protein content of the infant formula may be calculated by any method known to those of skill in the art. Suitably, the protein content may be determined by a nitrogen-to-protein conversion method. For example, as described in Maubois, J.L. and Lorient, D., 2016. Dairy science & technology, 96(1), pp.15-25. Preferably the protein content is calculated as nitrogen content × 6.25, as defined in European Commission Regulation (EU) 2016/127 of 25 September 2015. The nitrogen content may be determined by any method known to those of skill in the art. For example, nitrogen content may be measured by the Kjeldahl method.

### Protein concentration

eHFs typically contain 2.6-2.8 g protein per 100 kcal and AAFs typically contain 2.8-3.1 g protein per 100 kcal, to cover the needs of infants suffering gastrointestinal pathologies with severe malabsorption or infants requiring more proteins and calories to cover a higher metabolic rate.

The inventors have surprisingly shown that an eHF or an AAF with a lower protein content may support appropriate growth and development of allergic infants. Moreover, the inventors have surprisingly shown that the formula was safe and well-tolerated.

Accordingly, the eHF of the present invention comprises about 2.4 g or less protein per 100 kcal, or the AAF of the present invention comprises about 2.9g or less protein per 100kcal, preferably 2.8g or less protein per 100kcal. For example, the infant formula of the present invention may comprise about 2.3 g or less protein per 100 kcal, 2.25 g or less protein per 100 kcal, or 2.2 g or less protein per 100 kcal.

Suitably, the infant formula comprises about 1.8 g or more protein per 100 kcal. For example, the infant formula of the present invention may comprise about 1.86 g or more protein per 100 kcal, 1.9 g or more protein per 100 kcal, 2.0 g or more protein per 100 kcal, or 2.1 g or more protein per 100 kcal. Preferably, the infant formula comprises about 1.86 g or more protein per 100 kcal, in line with present EU regulations (EFSA NDA Panel, 2014. EFSA journal, 12(7), 3760).

The eHF of the present invention may comprise 1.8-2.4 g protein per 100 kcal, 1.86-2.4g protein per 100 kcal, 1.9-2.4 g protein per 100 kcal, 2.0-2.4 g protein per 100 kcal, 2.0-2.3 g protein per 100 kcal, 2.1-2.3 g protein per 100 kcal, or 2.15-2.25 g protein per 100 kcal.

The AAF of the present invention may comprise 1.8-2.9 g protein per 100 kcal, 1.9-2.8g protein per 100 kcal, 2.0-2.7 g protein per 100 kcal, 2.0-2.6 g protein per 100 kcal, 2.0-2.5 g protein per 100 kcal, 2.0-2.4 g protein per 100 kcal, 2.1-2.3 g protein per 100 kcal, or 2.15-2.25 g protein per 100 kcal.

Preferably the infant formula of the present invention comprises between 2.0 and 2.4 g protein per 100 kcal, for example 2.1-2.3 g protein per 100 kcal, or 2.15-2.25 g protein per 100 kcal Preferably, the infant formula comprises about 2.2 g protein per 100 kcal.

### Protein source

The source of protein may be any source suitable for use in an infant formula. Suitably, the protein is cow's milk protein.

An extensively hydrolysed/hydrolysed whey-based formula may be more palatable than an extensively hydrolysed/hydrolysed casein-based formula and/or the subject may only be sensitised to casein protein. Suitably, therefore, more than about 50%, more than about 60%, more than about 70%, more than about 80%, more than about 90%, or about 100% of the protein is whey protein. Preferably, the protein source is whey protein.

The whey protein may be a whey from cheese making, particularly a sweet whey such as that resulting from the coagulation of casein by rennet, an acidic whey from the coagulation of casein by an acid, or the acidifying ferments, or even a mixed whey resulting from coagulation by an acid and by rennet. This starting material may be whey that has been demineralized by ion exchange and/or by electrodialysis and is known as demineralised whey protein (DWP).

The source of the whey protein may be sweet whey from which the caseino-glycomacropeptide (CGMP) has been totally or partially removed. This is called modified sweet whey (MSW). Removal of the CGMP from sweet whey results in a protein material with threonine and trytophan contents that are closer to those of human milk. A process for removing CGMP from sweet whey is described in EP 880902.

The whey protein may be a mix of DWP and MSW.

In some embodiments, the amount of casein in the infant formula is undetectable, for example less than 0.2 mg/kg. The amount of casein may be determined by any method known to those of skill in the art.

### Degree of hydrolysis

In eHFs, the protein is "extensively hydrolysed", such that the eHFs may be tolerated by more than 90% of infants with CMPA.

Protein hydrolysates may have an extent of hydrolysis that is characterised by NPN/TN%, which refers to the non-protein nitrogen divided by the total nitrogen × 100. The non-protein nitrogen refers to amino nitrogen that is free to react with a reagent such as trinitrobenzenesulfonic acid (TNBS). NPN/TN% may be determined by any method known to those of skill in the art. For example, NPN/TN% may be measured as described in Adler-Nissen (Adler-Nissen, J. (1979) J. Agric. Food Chem. 27: 1256-1262). Suitably, the protein may have an NPN/TN% greater than 90%, greater than 95% or greater than 98%.

The extent of hydrolysis may also be determined by the degree of hydrolysis. The "degree of hydrolysis" (DH) is defined as the proportion of cleaved peptide bonds in a protein hydrolysate and may be determined by any method known to those of skill in the art. Suitably the degree of hydrolysis is determined by pH-stat, trinitrobenzenesulfonic acid (TNBS), o-phthaldialdehyde (OPA), trichloroacetic acid soluble nitrogen (SN-TCA), or formol titration methods. (Rutherfurd, S.M., 2010. Journal of AOAC International, 93(5), pp.1515-1522). The degree of hydrolysis (DH) of the protein can be more than 90, more than 95 or more than 98.

The extent of hydrolysis may also be determined by the peptide molecular mass distribution. The peptide molecular mass distribution may be determined by High performance size exclusion chromatography, optionally with UV detection (HPSEC/UV) (Johns, P.W., et al., 2011. Food chemistry, 125(3), pp.1041-1050). For example, the peptide molecular mass distribution may be a HPSEC peak area-based estimate determined at 205 nm, 214 nm or 220 nm. Suitably when the peptide molecular mass distribution is determined by HPSEC/UV, the "percentage of peptides by weight" that have a certain molecular mass may be estimated by the "fraction of peak area as a percentage of total peak area", that have the molecular mass, determined at 205 nm, 214 nm or 220 nm. Suitably, the extent of hydrolysis may be determined by the methods described in WO 2016/156077. Alternatively, the peptide molecular mass distribution may be determined by any method known to those of skill in the art, for example by sodium dodecyl sulphate-polyacrylamide gel electrophoresis (SDS-PAGE) (Chauveau, A., et al., 2016. Pediatric Allergy and Immunology, 27(5), pp.541-543).

Theoretically, to bind with cell membrane-bound IgE, peptides should be greater than about 1500 Da in size (approximately 15 amino acids) and to crosslink IgE molecules and to induce an immune response, they must be greater than about 3000 Da in size (approximately 30 amino acids) (Nutten, 2018. EMJ Allergy Immunol, 3(1), pp. 50-59).

Suitably, therefore, at least about 95%, at least about 98%, at least about 99% or about 100% of the peptides by weight in the eHF have a molecular mass of less than about 3000 Da. There may be no detectable peptides about 3000 Da or greater in size in the eHF.

Suitably, therefore, at least about 95%, at least about 98%, at least about 99% or about 100% of the peptides by weight in the eHF have a molecular mass of less than about 1500 Da. Preferably, at least 99% of the peptides by weight have a molecular mass of less than about 1500 Da. There may be no detectable peptides about 1500 Da or greater in size in the eHF.

Preferably, at least about 85%, at least about 90%, at least about 95%, at least about 98% or at least about 99% of the peptides by weight in the eHF have a molecular mass of less than about 1200 Da. More preferably, at least 95% or 98% of the peptides by weight in the eHF have a molecular mass of less than about 1200 Da.

Suitably, at least about 80%, at least about 85%, at least about 90%, or at least about 95% of the peptides by weight in the eHF have a molecular mass of less than about 1000 Da.

Preferably, at least about 95% of the peptides by weight in the eHF have a molecular mass of less than about 1000 Da.

Preferably, the eHF of the present invention has no detectable peptides about 3000 Da or greater in size; and at least about 95% of the peptides by weight have a molecular mass of less than about 1200 Da.

Having a high proportion of di- and tri-peptides may improve nitrogen (protein) absorption, even in patients with gut impairment. PEPT1 is a dedicated facilitator transport route for small peptide absorption (e.g. di- and tri-peptides). In the first weeks of life, intestinal PEPT1 is important for nutritional intake, and later for diet transition following weaning.

Thus, at least about 30%, at least about 40%, or at least about 50% of the peptides by weight in the eHF may be di- and tri-peptides. Preferably, at least about 45%, at least about 50%, 45-55%, or 50-54% of the peptides by weight in the eHF are di- and tri-peptides. More preferably, about 51-53%, or most preferably, about 52% of the peptides by weight in the eHF are di- and tri-peptides.

Suitably, at least about 30%, at least about 40%, or at least about 50% of the peptides by weight in the eHF have a molecular mass of between 240 and 600 Da. Preferably, at least about 45%, at least about 50%, 45-55%, or 50-54% of the peptides by weight in the eHF have a molecular mass of between 240 and 600 Da. More preferably, about 51-53%, or most preferably, about 52% of the peptides by weight in the eHF have a molecular mass of between 240 and 600 Da.

The peptides in the eHF may have a median molecular weight of 300Da to 370Da, preferably 320Da to 360Da.

The principal recognised cow's milk allergens are alpha-lactalbumin (aLA), beta-lactoglobulin (bLG) and bovine serum albumin (BSA).

Suitably, therefore, the eHF may have non-detectable aLA content, for example about 0.010 mg/kg aLA or less; the eHF may have non-detectable bLG content, for example about 0.010 mg/kg bLG or less; and/or the eHF may have non-detectable BSA content, for example about 0.010 mg/kg BSA or less. Preferably, the eHF of the invention comprises no detectable amounts of aLA, bLG and BSA. The content of aLA, bLG and BSA may be determined by any method known to those of skill in the art, for example ELISA.

In preferred embodiments, the eHF of the present invention: has no detectable peptides about 3000 Da or greater in size; at least about 95% of the peptides by weight have a molecular mass of less than about 1200 Da; optionally at least about 45%, at least about 50%, or 45-55% of the peptides by weight have a molecular mass of between 240 and 600 Da and/or are di- or tri-peptides; the eHF of the present invention comprises about 1 g/L 2'-fucosyllactose and about 0.5 g/L lacto-N-neotetraose and/or about 0.15 g/100kcal 2'-fucosyllactose and about 0.075 g/100kcal lacto-N-neotetraose; and the eHF comprises no added MCT.

### Method of hydrolysis

Proteins for use in the infant formula of the invention may be hydrolysed by any suitable method known in the art. For example, proteins may be enzymatically hydrolysed, for example using a protease. For example, protein may be hydrolysed using alcalase (e.g. at an enzyme:substrate ratio of about 1-15% by weight and for a duration of about 1-10 hours). The temperature may range from about 40°C to 60°C, for example about 55°C. The reaction time may be, for example, from 1 to 10 hours and pH values before starting hydrolysis may, for example, fall within the range 6 to 9, preferably 6.5 to 8.5, more preferably 7.0 to 8.0.

Porcine enzymes, in particular porcine pancreatic enzymes may be used in the hydrolysis process. For example, WO9304593 A1 discloses a hydrolysis process using trypsin and chymotrypsin, which includes a two-step hydrolysis reaction with a heat denaturation step in between to ensure that the final hydrolysate is substantially free of intact allergenic proteins. The trypsin and chymotrypsin used in these methods are preparations produced by extraction of porcine pancreas.

WO2016156077A1 discloses a process for preparing a milk protein hydrolysate comprising hydrolysing a milk-based proteinaceous material with a microbial alkaline serine protease in combination with bromelain, a protease from Aspergillus and a protease from Bacillus.

### Free amino acids

The infant formula of the invention may comprise free amino acids.

The levels of free amino acids may be chosen to provide an amino acid profile that is sufficient for infant nutrition, in particular an amino acid profile that satisfies nutritional regulations (e.g. European Commission Directive 2006/141/EC).

For example, free amino acids may be incorporated in the eHF of the invention to supplement the amino acids comprised in the peptides.

In AAF the protein content of the infant formula is provided by free amino acis.

Example free amino acids for use in the infant formula of the invention include histidine, isoleucine, leucine, lysine, methionine, cysteine, phenylalanine, tyrosine, threonine, tryptophan, valine, alanine, arginine, asparagine, aspartic acid, glutamic acid, glutamine, glycine, proline, serine, carnitine, taurine and mixtures thereof.

Suitably, therefore, the free amino acids in the eHF may be present in a concentration of 50% or less, 40% or less, 30% or less, or 25% or less by weight based on the total weight of amino acids. Preferably, the eHF comprises 25% or less by weight of free amino acids based on the total weight of amino acids. More preferably, the free amino acids in the eHF are present in a concentration of 20-25%, 21-23%, or about 22% by weight based on the total weight of amino acids.

The free amino acids content may be determined by any method known of skill in the art. Suitably, the free amino acids content may be obtained by separation of the free amino acids present in an aqueous sample extract by ion exchange chromatography and photometric detection after post-column derivatization with ninhydrin reagent. Total amino acids content may be obtained by hydrolysis of the test portion in 6 mol/L HCl under nitrogen and separation of individual amino acids by ion-exchange chromatography, as describe above.

In preferred embodiments, the eHF of the present invention: has no detectable peptides about 3000 Da or greater in size; at least about 95% of the peptides by weight have a molecular mass of less than about 1200 Da; optionally at least about 45%, at least about 50%, or 45-55% of the peptides by weight have a molecular mass of between 240 and 600 Da and/or are di- or tri-peptides, and/or 20-25%, 21-23%, or about 22% by weight based on the total weight of amino acids; the eHF of the present invention comprises about 1 g/L 2'-fucosyllactose and about 0.5 g/L lacto-N-neotetraose and/or about 0.15 g/100kcal 2'-fucosyllactose and about 0.075 g/100kcal lacto-N-neotetraose; and the eHF comprises no added MCT..

### Carbohydrate

The carbohydrate content of the infant formula of the invention is preferably in the range 9-14 g carbohydrate per 100 kcal.

The carbohydrate may be any carbohydrate which is suitable for use in an infant formula.

Example carbohydrates for use in the infant formula of the invention include lactose, saccharose, maltodextrin and starch. Mixtures of carbohydrates may be used.

In one embodiment, the carbohydrate content comprises maltodextrin. In one embodiment, at least about 20%, at least about 25%, at least about 30%, at least about 35%, at least about 40%, at least about 50%, at least about 60% or at least about 70% by weight of the total carbohydrate content is maltodextrin.

In one embodiment, the carbohydrate content comprises lactose. In one embodiment, at least about 20%, at least about 25%, at least about 30%, at least about 35%, at least about 40%, at least about 50%, at least about 60% or at least about 70% by weight of the total carbohydrate content is lactose.

In one embodiment, the carbohydrate comprises lactose and maltodextrin.

### Fat

The fat content of the infant formula of the invention is preferably in the range 4.0-6.0 g fat per 100 kcal.

The fat may be any lipid or fat which is suitable for use in an infant formula.

Example fats for use in the infant formula of the invention include sunflower oil, low erucic acid rapeseed oil, safflower oil, canola oil, olive oil, coconut oil, palm kernel oil, soybean oil, fish oil, palm oleic, high oleic sunflower oil and high oleic safflower oil, and microbial fermentation oil containing long chain, polyunsaturated fatty acids.

The fat may also be in the form of fractions derived from these oils, such as palm olein, medium chain triglycerides (MCT) and esters of fatty acids such as arachidonic acid, linoleic acid, palmitic acid, stearic acid, docosahexaeonic acid, linolenic acid, oleic acid, lauric acid, capric acid, caprylic acid, caproic acid, and the like.

Further example fats include structured lipids (i.e. lipids that are modified chemically or enzymatically in order to change their structure). Preferably, the structured lipids are sn2 structured lipids, for example comprising triglycerides having an elevated level of palmitic acid at the sn2 position of the triglyceride. Structured lipids may be added or may be omitted.

Oils containing high quantities of preformed arachidonic acid (ARA) and/or docosahexaenoic acid (DHA), such as fish oils or microbial oils, may be added.

Long chain polyunsaturated fatty acids, such as dihomo-γ-linolenic acid, arachidonic acid (ARA), eicosapentaenoic acid and docosahexaenoic acid (DHA), may also be added.

The infant formula may comprise 2-20 mg ARA per 100 kcal, 5-15 ARA per 100 kcal, or about 10 mg ARA per 100 kcal and/or 2-20 mg DHA per 100 kcal, 5-15 DHA per 100 kcal, or about 10 mg DHA per 100 kcal. Preferably, the infant formula comprises about 10 mg ARA per 100 kcal and about 10 mg DHA per 100 kcal.

### Medium chain triglycerides (MCTs)

A high concentration of MCT may impair early weight gain. MCT is not stored and does not support fat storage. For instance, Borschel et al. have reported that infants fed formula without MCT gained significantly more weight between 1-56 days than infants fed formulas containing 50% of the fat from MCT (Borschel, M., et al., 2018. Nutrients, 10(3), p.289).

Thus, about 30% or less by weight of the fat may be medium chain triglycerides (MCTs) in the infant formula of the present invention.

In some embodiments, about 25% or less by weight, 20% or less by weight, 15% or less by weight, 10% or less by weight, 5% or less by weight, 4% or less by weight, 3% or less by weight, 2% or less by weight, 1% or less by weight, 0.5% or less by weight, or 0.1% or less by weight of the fat is medium chain triglycerides (MCTs).

In some embodiments, 0-30% by weight, 0-25% by weight, 0-20% by weight, 0-15% by weight, 0-10% by weight, 0-5% by weight, 0-4% by weight, 0-3% by weight, 0-2% by weight, 0-1% by weight, 0-0.5% by weight, or 0-0.1% by weight of the fat is medium chain triglycerides (MCTs).

Preferably, the infant formula comprises no added MCTs. Suitably, about 0% by weight of the fat is MCTs and/or the infant formula comprises no detectable MCTs. Suitably, the infant formula comprises no MCTs.

In preferred embodiments, the eHF of the present invention: has no detectable peptides about 3000 Da or greater in size; at least about 95% of the peptides by weight have a molecular mass of less than about 1200 Da; 45-55% of the peptides by weight have a molecular mass of between 240 and 600 Da; free amino acids are present in a concentration of 20-25% by weight based on the total weight of amino acid; and the eHF comprises no added MCT.

### Further ingredients

The infant formula of the invention preferably also contains all vitamins and minerals understood to be essential in the daily diet in nutritionally significant amounts. Minimum requirements have been established for certain vitamins and minerals.

Example vitamins, minerals and other nutrients for use in the infant formula of the invention include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine and L-carnitine. Minerals are usually added in their salt form.

The infant formula of the invention may comprise one or more carotenoids.

The infant formula of the invention may also comprise at least one probiotic. The term "probiotic" refers to microbial cell preparations or components of microbial cells with beneficial effects on the health or well-being of the host. In particular, probiotics may improve gut barrier function.

Preferred probiotics are those which as a whole are safe, are L(+) lactic acid producing cultures and have acceptable shelf-life for products that are required to remain stable and effective for up to 24 months.

Examples of probiotic micro-organisms for use in the infant formula of the invention include yeasts, such as *Saccharomyces, Debaromyces, Candida, Pichia* and *Torulopsis;* and bacteria, such as the genera *Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus* and *Lactobacillus.*

Specific examples of suitable probiotic microorganisms are: *Saccharomyces cereviseae, Bacillus coagulans, Bacillus licheniformis, Bacillus subtilis, Bifidobacterium bifidum, Bifidobacterium infantis, Bifidobacterium longum, Enterococcus faecium, Enterococcus faecalis, Lactobacillus acidophilus, Lactobacillus alimentarius, Lactobacillus casei* subsp. *casei, Lactobacillus casei Shirota, Lactobacillus curvatus, Lactobacillus delbruckii* subsp. *lactis, Lactobacillus farciminus, Lactobacillus gasseri, Lactobacillus helveticus, Lactobacillus johnsonii, Lactobacillus rhamnosus* (*Lactobacillus GG*), *Lactobacillus sake, Lactococcus lactis, Micrococcus varians, Pediococcus acidilactici, Pediococcus pentosaceus, Pediococcus acidilactici, Pediococcus halophilus, Streptococcus faecalis, Streptococcus thermophilus, Staphylococcus carnosus* and *Staphylococcus xylosus.*

The infant formula of the invention may also contain other substances which may have a beneficial effect such as prebiotics, lactoferrin, fibres, nucleotides, nucleosides and the like.

### Reduced occurrence, prevention and treatment of infections

The infant formula of the present invention may be used to reduce the occurrence of an infection and/or prevent an infection in an infant.

The infant formula of the present invention may also be used to treat an infection in an infant.

As used herein, "reduce the occurrence" of an infection means that the infant formula reduces the likelihood of infection and/or at least one symptom associated with infection.

As used herein, "prevent" an infection means that the infant has not yet contracted an infection and/or is not showing any symptoms of infection, and the infant formula prevents infection and/or prevents or at least one symptom associated with infection.

As used herein, "treating" an infection means that the infant has an infection, and the infant formula lessens, reduces, or improves at least one symptom associated with the infection and/or slow downs, reduces, or eliminates the infection.

The term "infant" refers to a child under the age of 12 months, for example a child between 0 and 6 months of age. Preferably, the infant has cow's milk protein allergy.

In one aspect the invention provides a method of reducing the occurrence of, preventing, or treating an infection in an infant, comprising administering to the infant an infant formula according to the present invention. In another aspect the invention provides an infant formula according to the present invention for use in reducing the occurrence of, preventing, or treating an infection in an infant.

As used herein "infection" refers to the invasion of an infant's body tissues by any pathogen, including viruses, bacteria, fungi, parasite and arthropods. Preferably, the infection is a bacterial or viral infection. The infection may be an ear infection, a respiratory infection, a gastrointestinal infection and/or a urinary tract infection. Preferably, the infection is respiratory infection and/or an ear infection. Most preferably, the infection is an ear infection.

An ear infection may be an outer, middle, and/or inner ear infection. Preferably, the ear infection is a middle ear infection (otitis media). Otitis media (OM) is one of the most common childhood infections, the leading cause of doctors' visits by children, and the most frequent reason children consume antibiotics or undergo surgery in developed countries. *Streptococcus pneumoniae, Moraxella catarrhalis,* and non-typeable *Haemophilus influenza* are the predominant bacterial pathogens that cause OM. Viruses are an increasing cause of OM, alone or with bacterial co-pathogens. Many respiratory viruses contribute, but viral OM is most frequently caused by respiratory syncytial virus or rhinovirus (Rovers, M.M., et al., 2004. Otitis media. The lancet, 363(9407), pp.465-473).

The ear infection may be acute OM (AOM) or OM with effusion (OME), which are different stages of the OM continuum. AOM is defined as the presence of middle-ear effusion (MEE) in conjunction with the rapid onset of one or more signs or symptoms of inflammation in the middle ear, such as otalgia or pulling of the ear, otorrhoea, fever, or irritability. OME is defined as MEE without signs or symptoms of an acute infection. Some children manifest non-infectious discomfort, including hearing loss, irritability, clumsiness, or sleep disruption (Rovers, M.M., et al., 2004. Otitis media. The lancet, 363(9407), pp.465-473).

Accordingly, the infant formula of the invention can be used to reduce the occurrence of, prevent, or treat AOM. In another embodiment the formula of the invention can be used to reduce the occurrence of, prevent, or treat an OME. In another embodiment, the formula of the invention can be used to reduce the occurrence of, prevent, or treat MEE, otalgia or pulling of the ear, otorrhoea, fever, irritability, hearing loss, clumsiness, or sleep disruption.

In some embodiments the infection is a respiratory infection. The disease burden from respiratory infection is thought to be greater than that of any other cause of disease. In 2002, 18% of mortality for children younger than 5 years of age was caused by respiratory infections. A respiratory infection may be an upper respiratory tract infection (URTI) and/or a lower respiratory tract infection (LRTI) (Tregoning, J.S. and Schwarze, J., 2010. Clinical microbiology reviews, 23(1), pp.74-98).

URTI may be characterized by the following clinical symptoms: nasal (running nose, stuffed nose, blowing nose, yellow secretion, bloody secretion, sneezing); and/or pharyngeal (scratchy throat, sore throat, hoarseness); with fever (> 38°C) and/or otitis.

LRTI may be characterized by the following clinical symptoms: pneumonia (an episode of pneumonia may be defined as the illness which is reported as cough or difficult breathing); and/or bronchial (cough and/or secretion and/or yellow secretion).

Accordingly, the infant formula of the invention can be used to reduce the occurrence of, prevent, or treat an infection URTI and/or LRTI and/or one or more of running nose, stuffed nose, blowing nose, yellow secretion, bloody secretion, sneezing, scratchy throat, sore throat, hoarseness, fever, otitis, pneumonia, or bronchitis.

The infant formula of the present invention may reduce the risk of an infant developing an infection described herein e.g., LTRI, UTRI and/or OM.

Reducing the occurrence of infant developing an infection may mean that the risk of an infant developing an infection when administered an infant formula according to the present invention is reduced compared to an infant administered a conventional infant formula, such as the control formula used in the examples.

In one embodiment, the risk of infection may be reduced from when the infant formula is first administered to an infant (V0), to 6 months after the infant formula is first administered to the infant (V6), preferably when the infant formula was administered to the infant for 4 months or more, e.g. from V0, to 4 months after the infant formula is first administered to the infant (V4). The risk of infection may also be reduced from V4 to V6.

The infant formula of the present invention may reduce the use of antipyretic and/or antibiotics as concomitant medications (e.g. during and/or after the period of administration of the infant formula). As used herein "antipyretics" are substances that reduce fever and will be well known to those of skill in the art. As used herein "antibiotics" are substances that are used to treat or prevent some types of bacterial infection and will be well known to those of skill in the art.

The inventors have surprisingly shown that an infant formula according to the present invention reduced the amount of concomitant medication. For example, the infant formula according to the present invention may reduce the amount of antipyretics used as concomitant medication. The infant formula according to the present invention may also reduce the amount of antibiotics used as concomitant medication

Accordingly, in another aspect the invention provides a method of reducing the amount of antipyretics used by an infant, comprising administering to the infant an infant formula according to the present invention. In another aspect the invention provides an infant formula according to the present invention for use in reducing the amount of antipyretics used by an infant.

The use of an infant formula according to the present invention preferably reduces the amount of medicament (e.g., antipyretic) required relative to the use of conventional infant formula, such as the control formula used in the examples.

### Cow's milk protein allergy and obesity

In addition to preventing, reducing the occurrence of, or treating an infection, the infant formula of the invention may prevent obesity and/or reduce the risk of obesity in an infant and/or prevent cow's-milk protein allergy in the infant.

Thus, in preferred aspects, the invention provides an infant formula according to the present invention for use in both (i) preventing, reducing the occurrence of, or treating an infection in an infant and (ii) preventing/treating obesity and/or reducing the risk of obesity in an infant. Additionally, the infant formula may be use in treating and/or preventing cow's-milk protein allergy (CMPA) and/or one or more symptom of CMPA.

Most children with CMP-allergy (CMPA) have two or more symptoms: 50-70% have skin symptoms, 50-60% have gastrointestinal symptoms, and 20-30% have airway symptoms. Severe and life-threatening symptoms may occur in 10% of children. (Nutten, 2018. EMJ Allergy Immunol, 3(1), pp. 50-59).

Skin symptoms can include urticaria, atopic eczema, and angioedema. Gastrointestinal symptoms can include dysphagia, frequent regurgitation, colic, abdominal pain, vomiting, anorexia, refusal to feed, diarrhea (with or without intestinal protein or blood loss), constipation (with or without perianal rash), failure to thrive, occult blood loss, and iron-deficiency anemia. Respiratory symptoms can include runny nose, wheezing, and chronic coughing. Other general symptoms include anaphylaxis and shock-like symptoms with severe metabolic acidosis, vomiting and diarrhoea (food protein-induced enterocolitis syndrome). These symptoms are typically unrelated to infections, drug intake, or other causes (Koletzko, S., et al., 2012. Journal of pediatric gastroenterology and nutrition, 55(2), pp.221-229).

Infants with CMPA may require higher levels of protein due to e.g. gastrointestinal symptoms. Thus, eHFs typically contain 2.6-2.8 g protein per 100 kcal and AAFs typically contain 2.8-3.1 g protein per 100 kcal, to cover the needs of infants suffering gastrointestinal pathologies with severe malabsorption or infants requiring more proteins and calories to cover a higher metabolic rate.

Consumption of higher protein infant formula has, however been associated with greater weight and body mass index at 2 years of age and higher circulating concentrations of plasma essential amino acids, insulin-like growth factor-1, and C-peptide, which can induce weight gain and adipogenic activity. Lower protein content may diminish the later risk of obesity (Totzauer, M., et al., 2018. Obesity, 26(7), pp.1203-1210).

For instance, the European Childhood Obesity Project (CHOP) has shown that infants fed infant formula containing high protein levels gained more weight during the first year of life and had a higher BMI and risk of obesity at the age of 6 years than those fed an infant formula containing lower levels of protein (Totzauer, M., et al., 2018. Obesity, 26(7), pp.1203-1210).

The present inventors have surprisingly shown that the infant formulas of the present invention supported appropriate growth and development of allergic infants. Moreover, the infant formula was safe and well-tolerated.

### Method of manufacture

The infant formula of the invention may be prepared in any suitable manner.

For example, the infant formula may be prepared by blending together the hydrolysed protein source, the carbohydrate source and the fat source in appropriate proportions. If used, the further emulsifiers may be included at this point. The vitamins and minerals may be added at this point but vitamins are usually added later to avoid thermal degradation. Any lipophilic vitamins, emulsifiers and the like may be dissolved in the fat source prior to blending. Water, preferably water which has been subjected to reverse osmosis, may then be mixed in to form a liquid mixture. Commercially available liquefiers may be used to form the liquid mixture. The liquid mixture may then be homogenised.

The liquid mixture may then be thermally treated to reduce bacterial loads. This may be carried out, for example, by means of steam injection, or using an autoclave or heat exchanger, for example a plate heat exchanger.

The liquid mixture may then be cooled and/or homogenised. The pH and solid content of the homogenised mixture may be adjusted at this point.

The homogenised mixture may then be transferred to a suitable drying apparatus such as a spray dryer or freeze dryer and converted to powder. If a liquid infant formula is preferred, the homogenised mixture may be sterilised, then aseptically filled into a suitable container or may be first filled into a container and then retorted.

### EXAMPLES

The invention will now be further described by way of Examples, which are meant to serve to assist one of ordinary skill in the art in carrying out the invention and are not intended in any way to limit the scope of the invention.

### Example 1 - Illustrative extensively hydrolysed infant formula

Below is an illustrative extensively hydrolysed infant formula according to the present invention. The eHF of the invention preferably contains all nutrients, vitamins and minerals understood to be essential in the daily diet in nutritionally significant amounts. Minimum requirements have been established for certain nutrients, vitamins and minerals.

| **Nutrient** | **Unit** | **per 100g** | **per 100kcal** | **per 100ml** |
|---|---|---|---|---|
| Energy | kcal | 506 | 100 | 67 |
| Fat | g | 26 | 5.1 | 3.4 |
| MCT | g | 0 | 0 | 0 |
| Available carbohydrates | g | 57 | 11 | 7.5 |
| Protein | g | 11.1 | 2.2 | 1.5 |
| 2'FL | g | 0.76 | 0.15 | 0.1 |
| LNnT | g | 0.38 | 0.075 | 0.05 |

### Example 2 - Safety and efficacy of extensively hydrolysed infant formula with reduced protein content.

### Study design

The Safety and efficacy of extensively hydrolysed infant formula with reduced protein content was investigated in a controlled, double blind, randomized, multi-center, interventional clinical trial of 2 parallel formula fed groups.

An objective of the clinical study was to show that infants with cow milk protein allergy (CMPA) and fed with a new eHF with reduced level of protein (Althéra 2.2) and with two Human Milk Oligosaccharides (HMOs) (Test Formula) have a growth in line with infants fed with commercial eHF (Althéra 2.5) without HMOs (Control Formula). The commercial eHF is currently approved as a food for special medical purposes (Regulation (EU) 2016/128). The primary study endpoint was therefore weight gain per day from enrolment to 4 months of follow-up with an age window at enrolment of 0 to 6 months (non-inferiority design).

Other objectives included assessing whether consumption of Test formula by CMPA infants (i) reduces medication use and risk for infections, such as ear infections, lower respiratory tract infections/morbidity, (ii) is well tolerated and allows for age appropriate growth and (iii) reduces health care costs. Secondary study endpoints therefore included: (1) Changes in other growth parameters, including weight-for-age, length-for-age, head circumference-for-age Z scores (WHO growth reference); (2) Safety (rates of adverse events and serious adverse effects); and (3) Events of interest (rates of infections, medication use).

The trial population was full-term infants with physician diagnosed CMPA as per standard clinical practice and with at least 2 symptoms per inclusion criterion. 130 infants completing 4 months of study formula intake were required.

The inclusion criteria were:
1. Full term infant (37 weeks ≤ gestation ≤ 42 weeks);
2. 2500g ≤ birth weight ≤ 4500g;
3. Having obtained the infant's parent's (or both parents' if required per country regulation) or legally authorized representative's (LAR) written informed consent;
4. Infant aged between birth and 6 months;
5. Exclusively formula-fed at time of enrolment or mothers of CMPA infant doing breastfeeding and independently elected before enrolment to exclusively formula feed; and
6. Infants with physician diagnosed (and untreated with extensively hydrolysed or amino acid infant formula) CMPA as per standard clinical practice and with at least 2 symptoms present from the following - Crying, Regurgitations, Liquid stools or Constipation, Skin atopic lesion, Urticaria or Respiratory symptoms. For diagnosis based on either a positive Ig E blood test, skin prick test, patch test or food challenge, only 1 symptom from above needs to be present.

The exclusion criteria were:
1. Congenital illness or malformation that may affect growth.
2. Demonstrated chronic malabsorption not due to CMPA.
3. Significant pre-natal and/or serious post-natal disease other than CMPA before enrolment (per investigator's medical decision).
4. Minor parent(s).
5. Infants whose parents or caregivers cannot be expected to comply with study procedures.
6. Currently participating or having participated in another clinical trial since birth.

The Test formula and the Control formula are shown below:

| | | **Test formula** | | | **Control formula** | | |
|---|---|---|---|---|---|---|---|
| **Nutrient** | **Unit** | **per 100g** | **per 100kcal** | **per 100ml** | **per 100g** | **per 100kcal** | **per 100ml** |
| Energy | kcal | 506 | 100 | 67 | 506 | 100 | 67 |
| Fat | g | 26 | 5.1 | 3.4 | 26 | 5.1 | 3.4 |
| MCT (Medium Chain Triglycerides) | g | 0 | 0 | 0 | 0 | 0 | 0 |
| Available Carbohydrates | g | 57 | 11 | 7.5 | 55.5 | 11 | 7.3 |
| Micronutrient/vitamin/ mineral mix | mg | 2350 | 465 | 310 | 2350 | 465 | 310 |
| 2'FL | g | 0.75 | 0.15 | 0.10 | - | - | - |
| LNnT | g | 0.38 | 0.075 | 0.05 | - | - | - |

In the Test formula and the Control formula:
- >99% by weight of the peptides had a molecular mass less than 3000 Da.
- >95% by weight of the peptides had a molecular mass less than 1200 Da.
- -52% by weight of the peptides were di- and tri-peptides (peptides with a molecular mass of 600-240 Da).
- -22.4% by weight of the total amino acids were free amino acids in the Test formula and Control formula.
- There was no detectable β-lactoglobulin (i.e. β-lactoglobulin content was less than 0.01 mg/kg).
- There was no detectable casein (i.e. casein content was less than 0.2 mg/kg).

Both formulas were in powder form, to be prepared as per instructions printed on the product label on the tins for oral intake by infants in amounts suitable for their weight, age and appetite.

Infants were given study formula until 4 month post-baseline at minimum (principal study period) and for as long as the infant requires per medical prescription (to maximum of 12 months of age).

Volume of feed required by the infant per day depended upon age, weight and appetite. Product was given *ad libitum* to the infant, although parents or caregivers followed guidelines printed on the label regarding appropriate volumes of feed to be offered per day and/or took advice from study staff.

Infants were attended up to 7 study visits: Baseline (enrolment), then every month until 4 months after baseline (+1, +2, +3, +4 months) and then 6 months after baseline. One additional final visit is planned when the infant will reach the age of 12 months old.

Randomization was a ratio of 1:1 per study formula group; and performed by minimization in Medidata balance. Stratification was by age at enrolment (0 to 60 days, 61 to 120 days, and above 120 days), gender, mode of delivery (vaginal or caesarean section). In case twins are enrolled, they were randomized to the same formula.

### Study results

The study showed that the Test formula supported appropriate growth and development of allergic infants, and was safe and well tolerated.

Primary analysis assessed whether infants fed with Test formula had a growth non-inferior to infants fed with Control formula over the 4 first months of treatment. Treatment difference and the one-sided simultaneous 97.5 % confidence interval was performed by a mixed model. Weight (kg) was modeled as a function of age (months), treatment, gender, age*treatment, age*gender. The model assumed a variance covariance matrix with autocorrelation type I structure for outcome at adjacent visit. The difference computed was used to estimate the treatment effect at 63 days [(14+112)/2]. A divisor of 0.0305 was used to rescale the estimate from kg/months to g/day. Key secondary analysis was done including for body weight, body length, and head circumference, stool characteristics, behavior patterns and healthcare resource use.

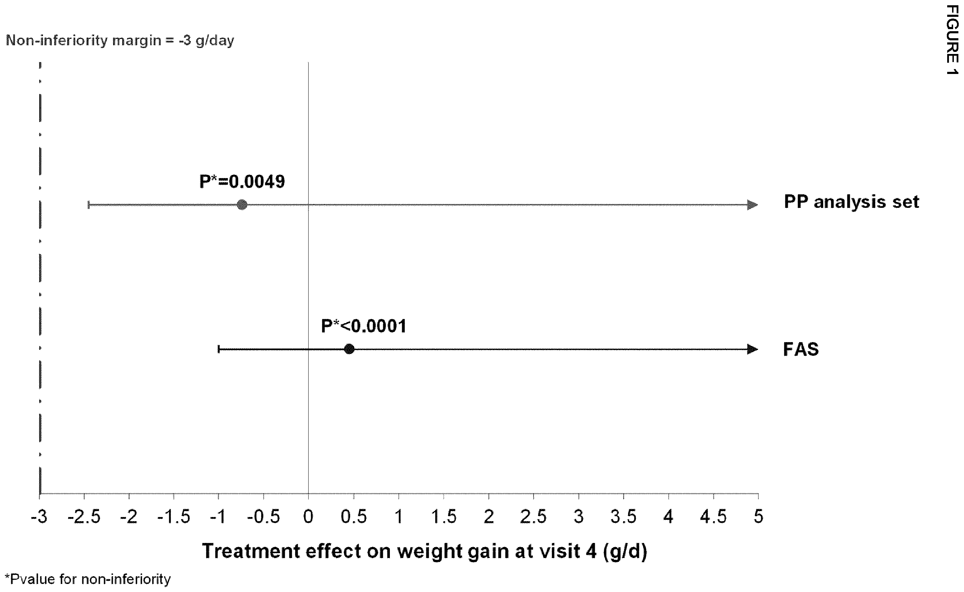

Infants receiving the Test formula with HMO and reduced protein content achieved normal growth, in line with to the WHO growth reference. In particular, primary analysis showed that weight gain [g/d] of infants receiving Test formula was non-inferior to growth with the Control formula (Figure 1). There were no significant differences in any of the anthropometric parameters at any of the time points up to the 4-month follow up.

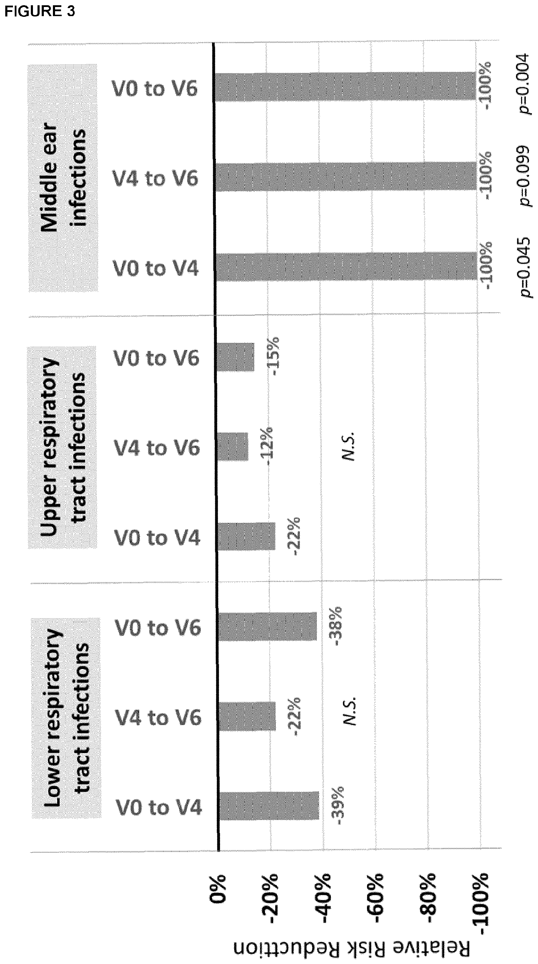

The safety profile of Test and Control formula were similar. The rate of otitis media/middle ear infection was significantly lower in the group fed Test formula between V0-V4 and V0-V6 (Figure 3).

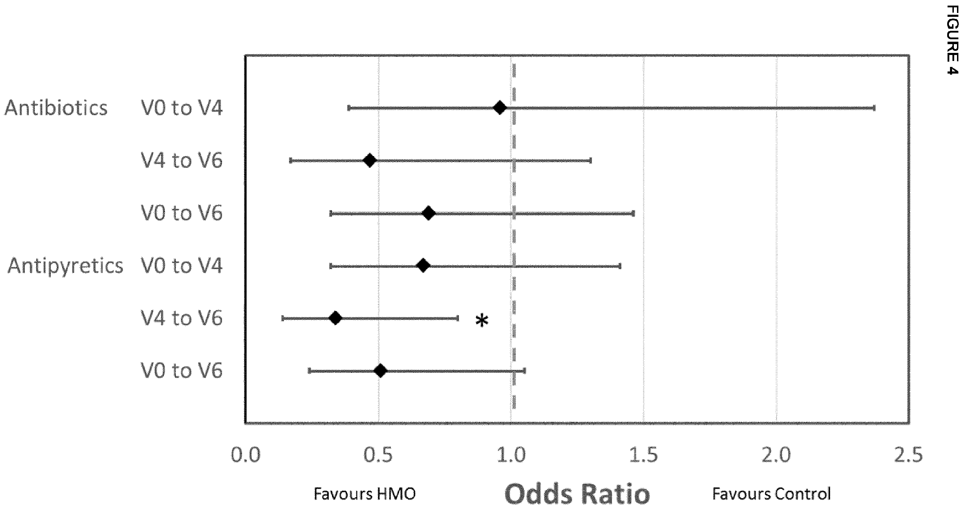

There was also a non-significant relative risk reduction in lower respiratory tract infections and upper respiratory tract infections (Figure 3). The use of antipyretics was significantly lower in the Test formula group between V4-V6 (Figure 4).

### Example 3 - Illustrative amino acid-based infant formula

Below is an illustrative amino acid-based infant formula according to the present invention. The AAF of the invention preferably contains all nutrients, vitamins and minerals understood to be essential in the daily diet in nutritionally significant amounts. Minimum requirements have been established for certain nutrients, vitamins and minerals.

| **Nutrient** | **Unit** | **per 100g** | **per 100kcal** | **per 100ml** |
|---|---|---|---|---|
| Energy | kcal | 503 | 100 | 70 |
| Fat | g | 25 | 5 | 3.5 |
| MCT | g | 0 | 0 | 0 |
| Available carbohydrates | g | 57 | 11.4 | 7.9 |
| Protein | g | 11 | 2.2 | 1.5 |
| 2'FL | g | 0.70 | 0.14 | 0.10 |
| LNnT | g | 0.36 | 0.07 | 0.05 |

All publications mentioned in the above specification are herein incorporated by reference. Various modifications and variations of the disclosed methods, cells, compositions and uses of the invention will be apparent to the skilled person without departing from the scope and spirit of the invention. Although the invention has been disclosed in connection with specific preferred embodiments, it should be understood that the invention as claimed should not be unduly limited to such specific embodiments. Indeed, various modifications of the disclosed modes for carrying out the invention, which are obvious to the skilled person are intended to be within the scope of the following claims.

**The invention also relates to the following embodiments**
1. An infant formula for use in reducing the occurrence of, or preventing, an infection in an infant, wherein the infant formula is an extensively hydrolysed infant formula (eHF) or an amino acid-based infant formula (AAF), wherein the infant formula comprises protein, carbohydrate and fat, wherein the eHF comprises about 2.4 g or less protein per 100 kcal, or the AAF comprises about 2.9 g or less protein per 100kcal, wherein the infant formula further comprises 2'-fucosyllactose (2'FL) and/or lacto-N-neotetraose (LNnT), and wherein about 30% or less by weight of the fat is medium chain triglycerides (MCTs).
2. A method of reducing the occurrence of, or preventing, an infection in an infant, wherein the method comprises administering to the infant an infant formula, wherein the infant formula is an eHF or an AAF, wherein the infant formula comprises protein, carbohydrate and fat, wherein the eHF comprises about 2.4 g or less protein per 100 kcal, or the AAF comprises about 2.9 g or less protein per 100kcal, wherein the infant formula further comprises 2'-fucosyllactose (2'FL) and/or lacto-N-neotetraose (LNnT), and wherein about 30% or less by weight of the fat is medium chain triglycerides (MCTs).
3. An infant formula for use according to embodiment 1, or a method according to embodiment 2, wherein the infant formula comprises 2'FL and LNnT
4. An infant formula for use according to embodiment 1 or 3, or a method according to embodiment 2 or 3, wherein the infant formula comprises 0.5-3 g/L, 0.8-1.5 g/L, or about 1 g/L 2'FL, preferably wherein the infant formula comprises about 1 g/L 2'FL.
5. An infant formula for use according to any one of embodiments 1, or 3-4, or a method according to any one of embodiments 2-4, wherein the infant formula comprises 0.2-1 g/L, 0.5-0.8 g/L, or about 0.5 g/L LNnT, preferably wherein the infant formula comprises about 0.5 g/L LNnT.
6. An infant formula for use according to any one of embodiments 1, or 3-5, or a method according to any one of embodiments 2-5, wherein the infant formula comprises about 1 g/L 2'FL and about 0.5 g/L LNnT.
7. An infant formula for use according to any one of embodiments 1, or 3-6, or a method according to any one of embodiments 2-6, wherein the infant formula is an eHF and wherein the infant formula comprises 1.8-2.4 g protein per 100 kcal, preferably between 2.0 and 2.4g protein per 100kcal, preferably 2.1-2.3 g protein per 100 kcal, or 2.15-2.25 g protein per 100 kcal.
8. An infant formula for use according to any one of embodiments 1, or 3-6, or a method according to any one of embodiments 2-6, wherein the infant formula is an AAF, and wherein the infant formula comprises 1.8-2.9g protein per 100kcal, preferably 1.9-2.8g protein per 100 kcal, preferably 2.0-2.7 g protein per 100kcal, more preferably 2.0-2.6 g protein per 100 kcal, or 2.0-2.4 g per 100kcal.
9. An infant formula for use according to any one of embodiments 1, or 3-8, or a method according to any one of embodiments 2-8, wherein the infant formula comprises about 2.2 g protein per 100 kcal.
10. An infant formula for use according to any one of embodiments 1, or 3-9, or a method according to any one of embodiments 2-9, wherein about, about 25% or less by weight, 20% or less by weight, 15% or less by weight, 10% or less by weight, 5% or less by weight, or 1% or less by weight of the fat in the infant formula is medium chain triglycerides (MCTs).
11. An infant formula for use according to any one of embodiments 1, or 3-10, or a method according to any one of embodiments 2-10, wherein the infant formula comprises no added MCTs.
12. An infant formula for use according to any one of embodiments 1, or 3-11, or a method according to any one of embodiments 2-11, wherein the infant formula comprises 9-14 g carbohydrate per 100 kcal and/or 4.0-6.0 g fat per 100 kcal.
13. An infant formula for use according to any one of embodiments 1, or 3-12, or a method according to any one of embodiments 2-12, wherein the infection is an ear infection, a respiratory infection, a gastrointestinal infection and/or a urinary tract infection.
14. An infant formula for use according to any one of embodiments 1, or 3-13, or a method according to any one of embodiments 2-13, wherein the infection is a respiratory infection, preferably an upper respiratory tract infection and/or a lower respiratory tract infection.
15. An infant formula for use according to any one of embodiments 1, or 3-13, or a method according to any one of embodiments 2-13, wherein the infection is an ear infection, preferably a middle ear infection.
16. An infant formula for use according to any one of embodiments 1, or 3-15, or a method according to any one of embodiments 2-15, wherein the infant has cow's milk protein allergy.

## Claims

1. An infant formula for use in reducing the occurrence of, or preventing, an infection in an infant, wherein the infant formula is an extensively hydrolysed infant formula (eHF) or an amino acid-based infant formula (AAF), wherein the infant formula comprises protein, carbohydrate and fat, wherein the eHF comprises about 2.4 g or less protein per 100 kcal, or the AAF comprises about 2.9 g or less protein per 100kcal, wherein the infant formula further comprises 2'-fucosyllactose (2'FL) and lacto-N-neotetraose (LNnT), and wherein about 30% or less by weight of the fat is medium chain triglycerides (MCTs), wherein the infant is intolerant or allergic to cow's milk protein.

2. An infant formula for use according to claim 1, wherein the infant formula comprises 0.5-3 g/L, 0.8-1.5 g/L, or about 1 g/L 2'FL, preferably wherein the infant formula comprises about 1 g/L 2'FL.

3. An infant formula for use according to any one of claims 1 to 2, wherein the infant formula comprises 0.2-1 g/L, 0.5-0.8 g/L, or about 0.5 g/L LNnT, preferably wherein the infant formula comprises about 0.5 g/L LNnT.

4. An infant formula for use according to any one of claims 1to 3, wherein the infant formula comprises about 1 g/L 2'FL and about 0.5 g/L LNnT.

5. An infant formula for use according to any one of claims 1 to 4, wherein the infant formula is an eHF and wherein the infant formula comprises 1.8-2.4 g protein per 100 kcal, preferably between 2.0 and 2.4g protein per 100kcal, preferably 2.1-2.3 g protein per 100 kcal, or 2.15-2.25 g protein per 100 kcal.

6. An infant formula for use according to any one of claims 1 to 4, wherein the infant formula is an AAF, and wherein the infant formula comprises 1.8-2.9g protein per 100kcal, preferably 1.9-2.8g protein per 100 kcal, preferably 2.0-2.7 g protein per 100kcal, more preferably 2.0-2.6 g protein per 100 kcal, or 2.0-2.4 g per 100kcal.

7. An infant formula for use according to any one of claims 1to 6, wherein the infant formula comprises about 2.2 g protein per 100 kcal.

8. An infant formula for use according to any one of claims 1to 7, wherein about, about 25% or less by weight, 20% or less by weight, 15% or less by weight, 10% or less by weight, 5% or less by weight, or 1% or less by weight of the fat in the infant formula is medium chain triglycerides (MCTs).

9. An infant formula for use according to any one of claims 1 to 8, wherein the infant formula comprises no added MCTs.

10. An infant formula for use according to any one of claims 1 to 9, wherein the infant formula comprises 9-14 g carbohydrate per 100 kcal and/or 4.0-6.0 g fat per 100 kcal.

11. An infant formula for use according to any one of claims 1 to 10, wherein the infection is an ear infection, a respiratory infection, a gastrointestinal infection and/or a urinary tract infection.

12. An infant formula for use according to any one of claims 1 to 11, wherein the infection is a respiratory infection, preferably an upper respiratory tract infection and/or a lower respiratory tract infection.

13. An infant formula for use according to any one of claims 1 to 12, wherein the infection is an ear infection, preferably a middle ear infection.

14. An infant formula for use according to any one of claims 1 to 13, wherein the infant has cow's milk protein allergy.
